# EUROPÄISCHE PATENTANMELDUNG

(11) **EP 1 935 368 A2**
(43) Veröffentlichungstag der Anmeldung: **25.06.2008**
(21) Anmeldenummer: 07000790.1
(22) Anmeldetag: 16.01.2007
(51) Int. Cl.: A61C 13/00, G06F 19/00

(54) **Verfahren betreffend den Transport von Zahnersatzteilen**

(30) Priorität: 22.12.2006 DE 102006061134
(71) Anmelder: Aepsilon Rechteverwaltungs GmbH, 82166 Grägelfing (DE)
(72) Erfinder: Holzner, Stephan, 80269 Hohenschäftlarn (DE); Weber, Gerhard, 86932 Pürgen (DE)
(74) Vertreter: Grünecker, Kinkeldey, Stockmair & Schwanhäusser Anwaltssozietät

(57) **Zusammenfassung**

Die Erfindung betrifft ein Verfahren, einen computerlesbaren Datenträger sowie einen Computer zum gemeinsamen Versenden oder Empfangen oder Verschicken von Daten für Zahnersatzteile und für Produktbestellungen sowie von Zahnersatzteilen oder Produkten.

## Beschreibung

Die Erfindung betrifft Verfahren, die sich mit dem Transport von Zahnersatzteilen beschäftigen.

Es ist bekannt, dass ein Dentallabor oder eine Zahnarztpraxis einen Modelldatensatz für die Herstellung eines Zahnersatzteils an ein Herstellungszentrum für Zahnersatzteile verschickt, letzteres das Zahnersatzteil herstellt und dieses in einem Paket, beispielsweise mit einem Paketdienst, an das Dentallabor oder die Zahnarztpraxis verschickt. Hierzu muss eine bestimmte Infrastruktur geschaffen werden, wie beispielsweise ein Computer mit einem Fernübertragungsanschluss sowie Einrichtungen, die den Versand von größeren Mengen von hergestellten Zahnersatzteilen möglichst automatisiert bewältigen können.

Dentallabors und Zahnarztpraxen haben daneben weiterhin gelegentlichen Materialbedarf, der üblicherweise durch Großhändler abgedeckt wird.

Aufgabe der vorliegenden Erfindung ist es, Verfahren, einen computerlesbaren Datenträger sowie einen Computer zur effizienteren Nutzung der Infrastruktur zur Versorgung von Zahnarztpraxen oder Dentallabors zu schaffen.

Diese Aufgabe wird gelöst mit dem Verfahren nach Anspruch 1, 6, 8 und 10, dem computerlesbaren Datenträger nach Anspruch 12 und dem Computer und/oder System nach Anspruch 14. Bevorzugte Ausführungsformen sind in den abhängigen Ansprüchen offenbart.

Bei einem Verfahren werden auf ein und demselben Computer die Datensätze, wie beispielsweise ein Modelldatensatz für ein Zahnersatzteil sowie ein Datensatz für die Bestellung von Produkten wie etwa Dentallabor- oder Zahnarztpraxenzubehör erstellt. Die beiden Datensätze können über die für den Computer vorgesehene Datenfernübertragungseinrichtung versandt werden. Bevorzugt ist es, hier die beiden Datensätze gleich zusammen zu versenden. Sie können jedoch auch nacheinander bzw. an verschiedene Stellen unabhängig voneinander versandt werden. Es wird jedoch vorzugsweise immer dieselbe Datenfernübertragungseinrichtung des einen Computers verwendet.

Die Datenfernübertragungseinrichtung kann beispielsweise einen Internetanschluss umfassen oder sein.

Besonders vorteilhaft ist ein Verfahren, bei dem der Datensatz für die Herstellung eines Zahnersatzteils bzw. während der Erstellung des ersten Datensatzes Informationen für die Erstellung des zweiten Datensatzes gesammelt bzw. generiert werden. So können bestimmte Zahnersatzteile beispielsweise einen bestimmten Zement oder Kleber erforderlich machen, insbesondere für den Fall, dass bei dem Zahnersatzteil mit besonderen Haftungsproblemen oder Stabilitätsproblemen zu rechnen sein wird, so dass bestimmte Spezialzemente oder Spezialkleber zum Einsatz kommen sollten. Die Information über derartige benötigte oder empfohlene Stoffe, wie Kleber, Zemente oder ähnliches Zubehör kann mit Hilfe der Information des ersten Datensatzes bzw. dem Schritt des Erstellens des ersten Datensatzes gewonnen werden und entsprechend für einen zweiten Datensatz aufbereitet werden.

Bei dem Erstellen eines Modelldatensatzes für die Herstellung eines Zahnersatzteils kann beispielsweise auch das Volumen bzw. die Fläche, für die Zement oder Kleber benötigt wird, ermittelt werden. Dementsprechend kann eine benötigte Menge von Kleber oder Zement errechnet werden und diese Information in den zweiten Datensatz eingearbeitet werden, so dass dann beispielsweise eine entsprechende Menge an Kleber oder Zement portioniert oder eine geeignete vorportionierte Menge ausgewählt und dem Dentallabor bzw. der Zahnarztpraxis zugesandt wird. Hierzu kann beispielsweise auf die Daten des ersten Datensatzes zugegriffen werden, wenn die Daten des zweiten Datensatzes erstellt werden.

Die Daten des ersten Satzes und die Daten des zweiten Satzes werden vorzugsweise nicht nur auf ein und demselben Computer, sondern auch mit ein und derselben Software erstellt. Dies erleichtert eine mögliche Verzahnung der Softwarekomponenten für die Erstellung der beiden Datensätze bzw. erlaubt auch eine entsprechende effiziente Ressourcennutzung der Computerressourcen.

Bei dem Erstellen des zweiten Datensatzes wird vorzugsweise auf einen elektronischen Produktkatalog zurückgegriffen. Der Produktkatalog kann auf dem Computer gespeichert sein, auf dem die beiden Datensätze erstellt werden. Er kann jedoch auch an einer anderen Stelle gespeichert sein, so dass auf den Produktkatalog per Datenfernübertragung zugegriffen werden kann. Dies erlaubt eine zentrale Erstellung und Aktualisierung eines entsprechenden Produktkatalogs. Der Katalog kann mehr als zehn, hundert, tausend, zehntausend oder hunderttausend Produkte umfassen.

Ein weiteres Verfahren betrifft den Empfang von Datensätzen und deren entsprechende Verarbeitung. Das Verfahren umfasst den Schritt des Empfangens eines ersten Datensatzes von einem Anwender, der per Datenfernübertragung übermittelt wird und der für die Herstellung von Zahnersatzteilen benötigt wird. Dieser Datensatz oder ein Teil davon oder ein daraus gewonnener Datensatz kann an ein Zahnersatzteilherstellungssystem weitergeleitet werden. Weiterhin wird ein zweiter Datensatz des Anwenders empfangen, wobei dieser zweite Datensatz den Versand von Produkten, wie etwa Zubehör für ein Dentallabor oder eine Zahnarztpraxis, betrifft. Ein entsprechender Datensatz wird an ein Produktversandsystem weitergeleitet. Die beiden Datensätze können als eine Einheit übermittelt werden und erst nach dem Empfangen dieser Einheit in die beiden Datensätze aufgeteilt werden. Die beiden Datensätze können aber auch getrennt voneinander von demselben Anwender versandt worden sein und entsprechend getrennt empfangen werden.

Das Weiterleiten des zweiten Datensatzes oder von daraus gewonnenen Daten an das Produktversandsystem kann mittels Datenfernübertragung erfolgen, wobei die Daten dann in der Regel an eine externe Firma übertragen werden. Das Weiterleiten kann jedoch auch lokal, wie in einem lokalen Netzwerk, innerhalb einer Firma erfolgen, falls das Produktversandsystem von derselben Firma betrieben wird, wie das Zahnersatzteilherstellungssystem.

Weiterhin betrifft die Erfindung ein Verfahren, bei dem zwei Datensätze des ein und desselben Anwenders empfangen werden, wobei ein Datensatz die Herstellung von Zahnersatzteilen betrifft und der andere den Versand von Produkten betrifft. Beide Datensätze werden in einem Zahnersatzteilherstellungszentrum empfangen. Weiterhin wird das Zahnersatzteil hergestellt und das Zahnersatzteil zusammen mit dem einen oder mehreren Produkten versandt. Da Zahnersatzteile ohnehin entsprechend von einem Zahnersatzteilherstellungszentrum versandt werden, gleichzeitig jedoch ein praktisch zu vernachlässigendes Gewicht haben, kann eine entsprechende Sendung zusätzlich für den Versand von weiteren Produkten, wie Dentallabor- oder Zahnarztpraxiszubehör, genutzt werden. Die für den Versand von solchen Zahnersatzteilen benötigte Infrastruktur kann auf diese Weise effizient genutzt werden. Dies gilt insbesondere für den Fall, dass in einem Zahnersatzteilherstellungszentrum beispielsweise mehrere hundert, tausend oder zehntausend Zahnersatzteile an einem einzigen Tag hergestellt und versandt werden.

Die in dem zweiten Datensatz angegebenen Produktdaten können von dem Zahnersatzteilherstellungszentrum an einen anderen Ort per Datenfernübertragung versandt werden und entsprechende Produkte von dort an das Zahnersatzteilherstellungszentrum geschickt werden. In diesem Fall kann das Zahnersatzteilherstellungszentrum eine größere Anzahl von Produkten aus mehreren zweiten Datensätzen auf einmal geliefert bekommen, so dass auch dies zu einer effizienten Ressourcennutzung führt.

Weiterhin betrifft die Erfindung ein Verfahren, bei dem ein Zahnersatzteil hergestellt wird, ein oder mehrere Produkte bereitgestellt werden und das Zahnersatzteil und die ein oder mehreren Produkte in eine gemeinsame Verpackung verpackt und diese eine Verpackung dann versandt wird. Aus den oben genannten Gründen führt dies zu einer effizienten Nutzung der Infrastruktur für das Versenden von Zahnersatzteilen.

Die Erfindung betrifft weiterhin einen computerlesbaren Datenträger mit Instruktionen zum Ausführen eines der oben genannten Verfahren mit einem Computer.

Der computerlesbare Datenträger kann Instruktionen für ein erstes Modul zur Gewinnung von Daten für die Herstellung eines Zahnersatzteils umfassen und Instruktionen für ein zweites Modul zur Gewinnung von Daten, die den Versand von Produkten betreffen. Die beiden Module sind entsprechende Softwaremodule, die vorzugsweise Module derselben Software sind.

Weiterhin betrifft die Erfindung einen Computer, der ausgebildet ist, eines der oben oder weiter unten aufgeführten Verfahren durchzuführen. Auch betrifft die Erfindung ein System, das beispielsweise mit solch einem Computer vorgesehen sein kann und das weiterhin über z.B. ein Zahnersatzteilherstellungssystem und/oder ein Zahnersatzteilverpackungssystem und/oder ein Zahnersatzteillagersystem und/oder ein Produktlagersystem und/oder ein Produktverpackungssystem und/oder ein System, das Verpackungen für die Abholung bereitstellt, umfassen. Das Produktlagersystem mit dem Produktverpackungssystem und dem System, das Verpackungen für die Abholung bereitstellt, stellt ein Produktversandsystem dar. Dieses System kann auch dazu vorgesehen sein, ein oder mehrere Zahnersatzteile mit zu lagern, zu verpacken bzw. zu versenden.

Bevorzugte Ausführungsformen der Erfindung sollen anhand der beiliegenden Figuren erläutert werden. Dabei zeigt:
- Fig. 1: eine Vorrichtung zum Erstellen von Datensätzen und
- Fig. 2 bis 5: schematische Darstellung von Abläufen bei der Durchführung von Ausführungsformen der Erfindung.

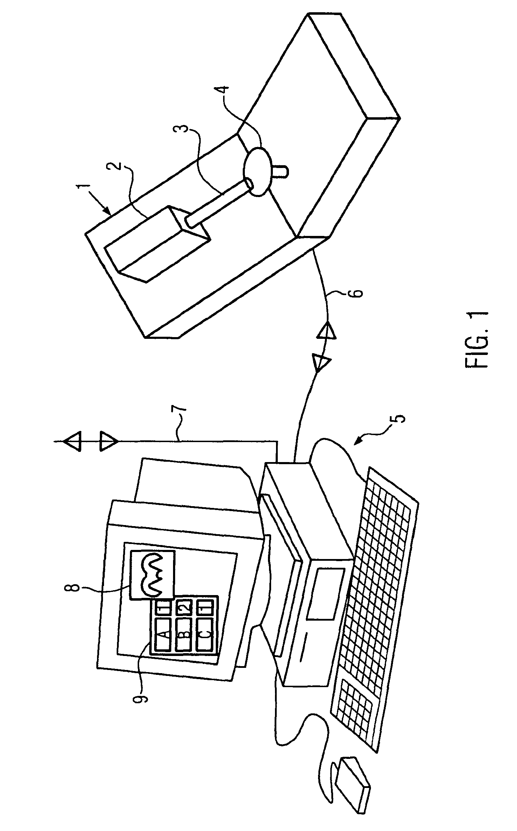

In Fig. 1 ist eine Vorrichtung 1 zum Scannen von Zahnmodellen bzw. Restzahnbereichsmodellen schematisch dargestellt. Derartige Vorrichtungen sind bekannt. Ein Modell kann beispielsweise auf einem Drehteller 4 angeordnet werden und von einer optischen Abtastvorrichtung 2 mit einem Lichtstrahl oder Lichtbündel 3 abgetastet werden.

Ein derartiger Scanner 1 ist mit einer lokalen Datenübertragungsverbindung 6 mit einem Computer 5 verbunden. Der Computer 5 steuert den Scanner 1 und speichert die beim Scannen gewonnenen Daten. Mit den Daten, die beim Scannen gewonnen wurden, kann ein Modell für ein Zahnersatzteil auf dem Computer 5 erstellt werden. Dies kann automatisch oder mit Unterstützung durch einen Anwender erfolgen. Ein entsprechendes Modell für ein Zahnersatzteil ist in dem Fenster 8 in Fig. 1 schematisch dargestellt.

In einem weiteren Fenster 9 in dem Computer in Fig. 1 ist schematisch eine Bestellliste für verschiedene Produkte A, B, C dargestellt. Von dem Produkt A soll eine Einheit, von dem Produkt B zwei Einheiten und von dem Produkt C eine Einheit bestellt werden. Es können auch nur jeweils ein Produkt oder zwei, vier, fünf oder mehr verschiedene Produkte bestellt werden können.

Ein Produktkatalog, aus dem die Produkte z.B. ausgewählt werden können, ist auf dem Computer 5 gespeichert (oder über ein lokales Netzwerk zugänglich). Der Produktkatalog kann aber auch bei dem Zahnersatzteilherstellungszentrum 14 oder dem Ort 26 gespeichert ein und über das Internet zugänglich gemacht sein.

Der Datensatz, der das im Fenster 8 dargestellte Zahnersatzteil betrifft, sowie ein Datensatz der Bestellung des Fensters 9, können über die gemeinsame Datenfernübertragungsleitung 7 verschickt werden. Dies kann beispielsweise ein Internetanschluss sein. Auch wenn in Fig. 1 eine Leitung dargestellt ist, kann die Verbindung auch drahtlos (über Funk) sein.

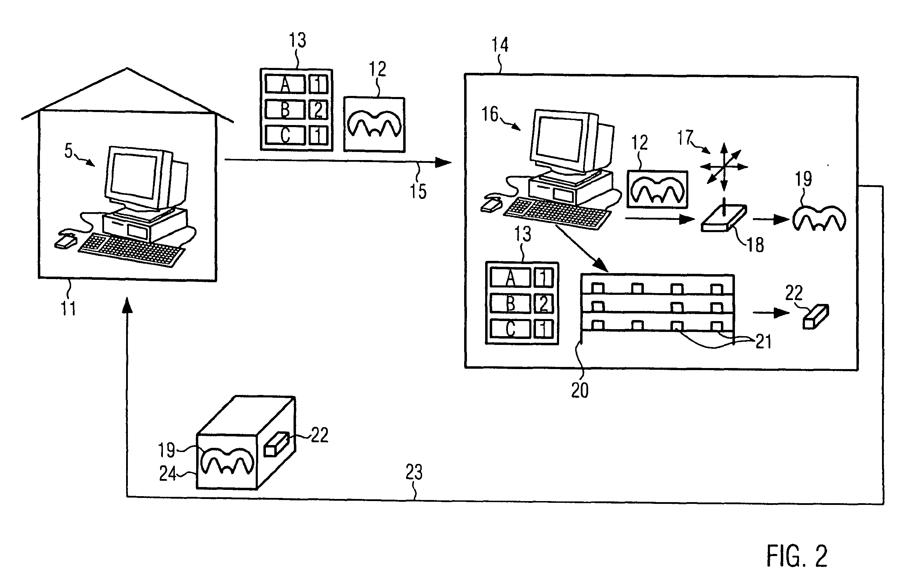

In Fig. 2 ist dargestellt, wie ein Computer 5 in einer Zahnarztpraxis 11 oder einem Dentallabor 11 angeordnet ist. Es ist ein Datensatz 12 symbolisch dargestellt, der das Modell eines Zahnersatzteils wiedergibt sowie eine Bestellliste 13. Diese werden per Datenfernübertragung 15 an ein Zahnersatzteilherstellungszentrum 14 versandt. Dort ist ein Computer 16 zum Empfang der mit der Datenfernübertragung 15 übertragenen Daten bereitgestellt. Der Computer 16 leitet Daten, die die Herstellung eines Zahnersatzteils betreffen, an ein Zahnersatzteilherstellungssystem 17 weiter. Dieses ist hier symbolisch durch eine Dreiachsfräsmaschine dargestellt. Es kann jedoch auch jedes andere beliebige Herstellungssystem für Zahnersatzteile sein. Hier ist insbesondere ein 3D-Laserlithografiesystem, eine mehr als dreiachsige Fräsmaschine oder jedes andere Rapidprototypingsystem möglich. Mit der in Fig. 2 symbolisch dargestellten Fräsmaschine 17 kann aus einem Rohling 18 ein Zahnersatzteil oder auch mehrere Zahnersatzteile gewonnen werden. Ein solches Zahnersatzteil ist in Fig. 2 unter der Bezugsziffer 19 gezeigt. Ein solches Zahnersatzteil 19 kann in einem (nicht dargestellten) Zahnersatzteillagersystem gelagert werden, bis es verpackt und/oder versandt wird.

Weiterhin wird ein Datensatz 13, der die Bestellung betrifft, an ein Produktlagersystem 20 bzw. ein Produktversandsystem 20 weitergeleitet, das in Fig. 2 schematisch als Hochregallager dargestellt ist. Dort sind Produkte 21 bereitgestellt, wobei in Fig. 2 ein aus dem Hochregallager 20 entnommenes Produkt 22 dargestellt ist. Das Zahnersatzteil 19 und das Produkt 22 können nun in einer gemeinsamen Verpackung 24 per Paketdienst 23 an das Dentallabor 11 oder die Zahnarztpraxis 11 versandt werden. Zum Verpacken des Zahnersatzteils und/oder des Produkts kann ein (nicht dargestelltes) Verpackungssystem vorgesehen sein, das vorzugsweise computergesteuert ist und automatisch arbeitet.

Das in Fig. 2 einzeln dargestellte Produkt 22 soll hier stellvertretend für ein oder mehrere Produkte stehen. Dies gilt entsprechend auch für die folgenden Abbildungen.

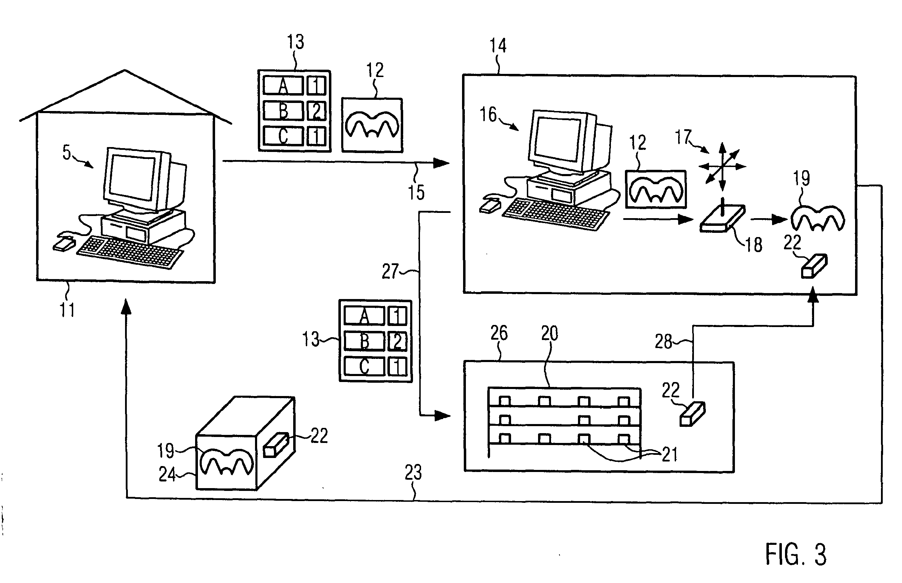

In Fig. 3 ist eine Variante des Verfahrens aus Fig. 2 dargestellt. Hier wird der zweite Datensatz 13 per Datenfernübertragung 27 an einen fernen Ort 26 übertragen. Dies kann beispielsweise der Ort eines Dentalzubehörgroßhändlers oder Ähnliches sein. Dieser liefert Produkte 22 per Produktversand, wie beispielsweise per Paketdienst 28, an das Zahnersatzteilherstellungszentrum 14. Auf diesem Wege können wiederum in einem gemeinsamen Paket 24 ein hergestelltes Zahnersatzteil 19 und ein Produkt 22 gemeinsam versandt werden.

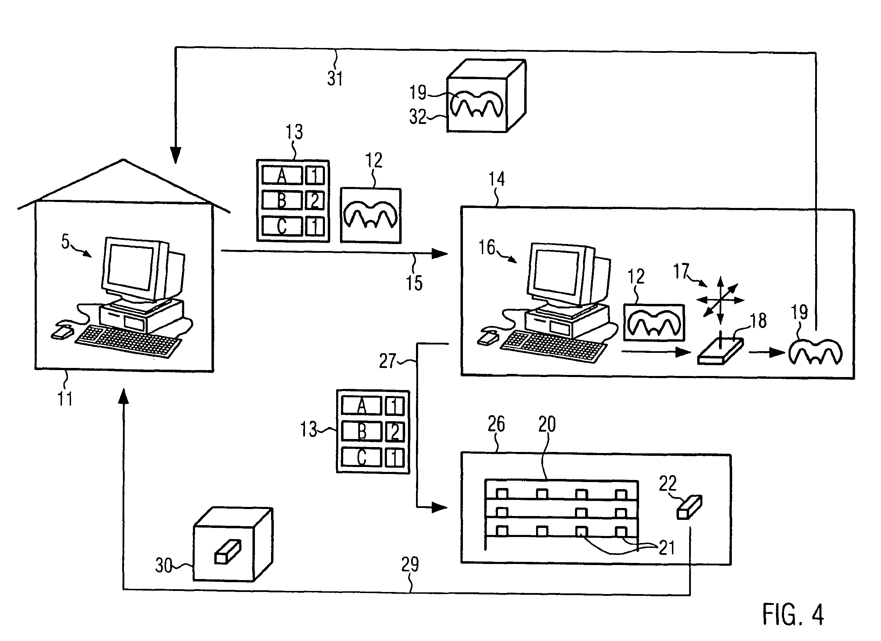

Eine Abwandlung des Verfahrens aus Fig. 3 ist in Fig. 4 dargestellt. Hier wird das von der Firma am Ort 26 bereitgestellte Produkt 22 nicht an das Zahnersatzteilherstellungszentrum 14 versandt, sondern direkt in einem eigenen Paket 30 an das Dentallabor bzw. die Zahnarztpraxis 11. Hier sind jeweilige getrennte Verpackungssysteme für Zahnersatzteil und Produkt vorgesehen.

Das Verfahren aus Fig. 3 und Fig. 4 kann auch wahlweise eingesetzt werden, beispielsweise ist das Verfahren nach Fig. 3 insbesondere für kleinere Produkte 22 vorteilhaft, wohingegen das Verfahren nach Fig. 4 für größere bzw. schwere Produkte vorteilhaft ist. Die Entscheidung, ob ein Produkt 22 nach dem Verfahren aus Fig. 3 oder nach dem Verfahren aus Fig. 4 an das Dentallabor bzw. die Zahnarztpraxis 11 versandt wird (entsprechend den beiden Varianten des Anspruchs 7), kann durch den Computer 16 in dem Herstellungszentrum 14 erfolgen.

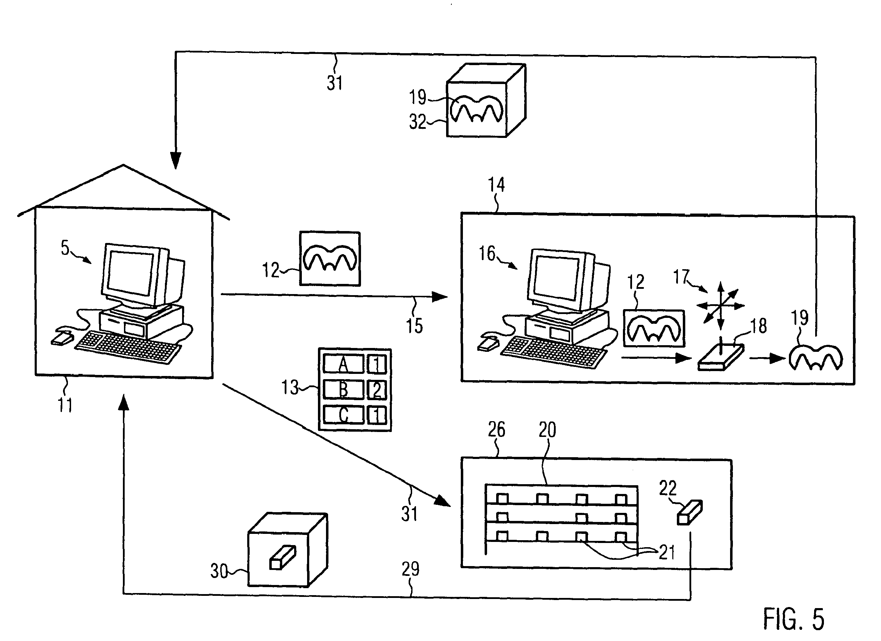

Fig. 5 zeigt eine weitere Variante eines Verfahrens, bei dem auf dem einen Computer 5 in der Zahnarztpraxis oder in dem Dentallabor 11 die Datensätze 12 und 13 erstellt werden, diese dann jedoch getrennt verschickt werden. Die Versendung des Datensatzes 12, der die Herstellung eines Zahnersatzteils betrifft, erfolgt an das Herstellungszentrum 14 und die des Datensatzes 13, der eine Bestellung von Produkten betrifft, erfolgt an den Ort 26. Die getrennte Versendung ist für den Anwender des Computers 5 jedoch vorzugsweise nicht sichtbar, sondern wird vorzugsweise vielmehr von ein und derselben Software durchgeführt.

In Fig. 5 ist dargestellt, dass ein Produkt 22 in einem Paket 30 zurück versandt wird und das Zahnersatzteil 19 in einem weiteren Paket 32.

Es ist hier jedoch auch möglich, dass das Zahnersatzteil 19 des Herstellungszentrums 14 an den Ort 26 versandt wird, so dass das Zahnersatzteil 19 in dem Paket 30 mitgeschickt wird bzw. ist es andersherum auch möglich, dass das Produkt 22 (stellvertretend für ein oder mehrere Produkte) an das Zahnersatzteilherstellungszentrum 14 geschickt wird und zusammen mit dem Zahnersatzteil 19 in dem Paket 32 versandt wird. Das Paket 30 kann dann entfallen.

Für den Fall, dass der zweite Datensatz nicht an das Zahnersatzteilherstellungszentrum 14 direkt versandt wird, ist es weiterhin vorteilhaft, wenn ein entsprechender Datensatz oder ein Teil davon oder ein daraus gewonnener Datensatz von dem Ort 26 an das Zahnersatzteilherstellungszentrum 14 übermittelt wird. Dies ermöglicht dann beispielsweise eine einheitliche Rechnungsstellung für das Zahnersatzteil 19 und das Produkt 22 durch das Zahnersatzteilherstellungszentrum 14.

## Patentansprüche

1. Verfahren mit den Schritten:
- Erstellen eines ersten Datensatzes (12), der für die Herstellung eines Zahnersatzteils (19) benötigt wird, mit einem Computer (5);
- Versenden des ersten Datensatzes (12) an ein Herstellungszentrum (14) für Zahnersatzteile (19) mittels Datenfernübertragung (15) durch den Computer (5),
**gekennzeichnet durch**
die Schritte:
- Erstellen eines zweiten Datensatzes (13), der den Versand von Produkten (22) betrifft, wie etwa Zubehör für ein Dentallabor oder eine Zahnarztpraxis, wie beispielsweise Zubehör für den Einbau von Zahnersatzteilen, mit dem Computer und
- Versenden des zweiten Datensatzes (13) an das Herstellungszentrum (14) vorzugsweise zusammen mit dem ersten Datensatz (12) oder an eine andere Stelle (26) als das Herstellungszentrum (14) per Datenfernübertragung (15).

2. Verfahren nach Anspruch 1, **dadurch gekennzeichnet, dass** bei dem Schritt des Erstellens des ersten Datensatzes (12) Information für die Erstellung des zweiten Datensatzes (13) gesammelt und/oder generiert wird, wie z.B. Information zu benötigten oder möglichen Stoffen für die Verwendung des zu dem ersten Datensatz (12) gehörenden Zahnersatzteils (19).

3. Verfahren nach Anspruch 1 oder 2, **dadurch gekennzeichnet, dass** bei dem Schritt des Erstellens des zweiten Datensatzes (13) auf Daten des ersten Datensatzes (12) zurückgegriffen wird.

4. Verfahren nach einem der Ansprüche 1 bis 3, **dadurch gekennzeichnet, dass** die beiden Datensätze (12,13) mit ein und derselben Software (8, 9) erstellt werden.

5. Verfahren nach einem der Ansprüche 1 bis 4, **dadurch gekennzeichnet, dass** bei dem Erstellen des zweiten Datensatzes (13) ein elektronischer Produktkatalog verwendet wird, wobei auf diesen Produktkatalog beispielsweise per Datenfernübertragung zugegriffen werden kann und dieser Produktkatalog bei dem Herstellungszentrum für Zahnersatzteile oder an anderer Stelle gespeichert ist.

6. Verfahren mit den Schritten:
- Empfangen eines ersten Datensatzes (12) von einem Anwender (11), der für die Herstellung von Zahnersatzteilen (19) benötigt wird und
- Weiterleiten des Datensatzes (12) oder zumindest eines Teils davon oder eines daraus gewonnenen Datensatzes an ein Zahnersatzteilherstellungssystem (17);
- Empfangen eines zweiten Datensatzes (13) von dem Anwender, wobei der zweite Datensatz (13) den Versand von Produkten (22) betrifft, wie etwa Zubehör für ein Dentallabor oder eine Zahnarztpraxis, wie beispielsweise Zubehör für den Einbau von Zahnersatzteilen und
- Weiterleiten des zweiten Datensatzes (13) oder zumindest eines Teils davon oder eines daraus gewonnenen Datensatzes an ein Produktlagersystem (22) und/oder ein Produktversandsystem (22).

7. Verfahren nach Anspruch 6, **dadurch gekennzeichnet, dass** das Weiterleiten an das Produktlagersystem (22) und/oder das Produktversandsystem (22) mittels Datenfernübertragung (27) oder mit lokalen Datenübertragungseinrichtungen wie einem lokalen Netzwerk erfolgt.

8. Verfahren mit den Schritten:
- Empfangen von:
- einem ersten Datensatz (12) von einem Anwender (11), der für die Herstellung von Zahnersatzteilen (19) benötigt wird und von
- einem zweiten Datensatz (13) von dem selben Anwender, der den Versand von einem oder mehreren Produkten (22) wie etwa Dentalzubehör betrifft in einem Zahnersatzteilherstellungszentrum (14)
- Herstellen des Zahnersatzteils (19) und
- Versenden (23) des Zahnersatzteils (19) zusammen mit dem einen oder mehreren Produkten (22).

9. Verfahren nach Anspruch 8, **dadurch gekennzeichnet, dass** Information betreffend den Versand von Produkten (22) wie etwa Dentalzubehör gemäß dem zweiten Datensatz (13) per Datenfernübertragung (27) an einen weiteren Ort (26), wie etwa den eines Dentalzubehörlieferanten versandt wird und entsprechendes Dentalzubehör (22) von dem weiteren Ort (26) an das Zahnersatzteilherstellungszentrum (14) geschickt (28) wird.

10. Verfahren mit den Schritten:
- Herstellen eines Zahnersatzteils (19);
- Bereitstellen von einem oder mehreren Produkten (22), wie etwa Zubehör für ein Dentallabor, beispielsweise Zubehör für den Einbau von Zahnersatzteilen und
- Verpacken des Zahnersatzteils (19) und der einen oder mehreren Produkte (22) in eine gemeinsame Verpackung (24).

11. Verfahren nach Anspruch 10, **gekennzeichnet durch** das Versenden der Verpackung (30).

12. Computerlesbarer Datenträger mit Instruktionen, die von einem Computer (5, 16) ausgeführt werden können, so dass ein Verfahren nach einem der Ansprüche 1 bis 10 durchgeführt wird.

13. Computerlesbarer Datenträger nach Anspruch 12, **dadurch gekennzeichnet, dass** Instruktionen für ein erstes Modul (8) zur Gewinnung von Daten für die Herstellung eines Zahnersatzteils und Instruktionen für ein zweites Modul (9) zur Gewinnung von Daten, die den Versand von Produkten, wie Dentallaborzubehör, wie etwa Zubehör für den Einbau von Zahnersatzteilen, betrifft, vorgesehen sind.

14. Computer und/oder System, der/das ausgebildet ist, eines der Verfahren nach einem der Ansprüche 1 bis 10 auszuführen.
